# EUROPEAN PATENT APPLICATION

(11) **EP 2 545 907 A1**
(43) Date of publication of application: **16.01.2013**
(21) Application number: 11382241.5
(22) Date of filing: 15.07.2011
(51) Int. Cl.: A61K 9/20, A61K 31/785

(54) **Aqueous wet granulation process for cross-linked polyallylamine polymers**

(71) Applicant: Combino Pharm, S.L., 08970 Sant Joan Despi, Barcelona (ES); Laboratorios Liconsa, S.A., 08028 Barcelona (ES)
(72) Inventor: Abascal Martínez, Natalia, E-08011 Barcelona (ES); Puigvert Colomer, Marina, E-08014 Barcelona (ES); Lloret Pérez, Sergio, E-08030 Barcelona (ES)
(74) Representative: ABG Patentes, S.L.

(57) **Abstract**

Wet granulation process for preparing cross-linked polyallylamine formulations comprising the steps of adding an aqueous granulating solvent system comprising at least 80% water by weight to a cross-linked polyallylamine, or a mixture of excipients comprising said cross-linked polyallylamine; granulating the mixture; and optionally drying the product obtained.

## Description

### Field of the invention

Process for making cross-linked polyallylamine polymer formulations by aqueous wet granulation techniques reducing or even avoiding the use of organic solvents. The cross-linked polyallylamine polymers according to the present invention are preferably selected from the group of Sevelamer, its derivatives and salts or mixtures thereof.

### Background of the invention

Hyperphosphatemia plays a role in the development of secondary hyperparathyroidism in renal insufficiency. Treatment of hyperphosphatemia includes reduction in dietary intake of phosphate, inhibition of intestinal phosphate absorption with phosphate binders, and removal of phosphate with dialysis.

Polyallylamine hydrochloride salts are produced by polymerization of allylamine hydrochloride in the presence of a radical initiator. Polyallylamine and polyallylamine hydrochloride are highly soluble in water, which upon cross linking with suitable N-alkylating reagents becomes water insoluble, and forms a water swellable polymer.

Cross-linked polyallylamine polymers are useful as active pharmaceutical ingredients for use in pharmaceutical compositions for treating hyperphosphatemia. A particularly interesting aliphatic amine polymer is Sevelamer, a polymeric phosphate binder intended for oral administration.

Sevelamer is a copolymer of prop-2-en-1-amine and epichlorohydrin. It is chemically known as poly(allylamine-co-*N,N'*-diallyl-1,3-diamino-2-hydroxypropane).

Sevelamer hydrochloride is a poly(allylamine hydrochloride) crosslinked with epichlorohydrin in which about forty percent of the amines are protonated. It is known chemically as poly(allylamime-co-N,N'-diallyl-1,3-diamino-2-hydroxypropane) hydrochloride. The reported structure of Sevelamer is represented below: 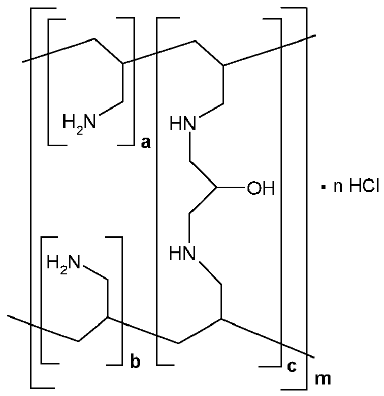
a, b = number of primary amine groups; a + b = 9
c = number of crosslinking groups; c = 1
n = fraction of protonated amines; n ∼ 0.4
m = large number to indicate extended polymer network.

Sevelamer hydrochloride is currently being marketed in the USA in the dosage form of 400 mg and 800 mg film-coated tablets for oral administration under the trade name RENAGEL®, for the treatment of patients with Chronic Kidney Disease (CKD) which are on hemodialysis. According to the prescribing information RENAGEL® is indicated for the control of serum phosphorus in such CKD patients.

Sevelamer carbonate is a polymeric amine which binds to phosphates when administered orally. Sevelamer carbonate has the same polymeric structure as Sevelamer hydrochloride in which carbonate replaces chloride as the counterion. It is known chemically as poly(allylamime-co-N,N'-diallyl-1,3-diamino-2-hydroxypropane) carbonate salt.

Sevelamer carbonate is developed by Genzyme Corporation and marketed in the USA in the dosage and form of 800 mg film-coated tablets and also of 800 mg and 2400 mg powder sachets for preparation of oral suspension, under the brand name RENVELA® for the control of serum phosphorus in patients with CKD on dialysis and not on dialysis.

Sevelamer salts bind the phosphorus in the gastrointestinal tract to facilitate phosphorus excretion in feces, thereby inhibiting phosphorus absorption from the gut and thereby lowering the plasma phosphorus concentration.

Sevelamer salts are insoluble but very hygroscopic and swell upon contact with water. Such swelling complicates formulating Sevelamer and its salts into a pharmaceutical composition.

Colesevelam hydrochloride is a modified cross-linked polyallylamine polymer N-alkylated with a specific ratio of (6-trimethylammonium)hexyl and decyl groups. Colesevelam hydrochloride has almost all of its amines protonated and contains about 21 % chloride by weight. It is developed by Genzyme and marketed in the USA under the brand name WELCHOL®. It is a non-absorbed, polymeric, lipid-lowering and glucose-lowering polymer which binds the bile acids in the intestine and impedes their reabsorption. It is administered orally and it is indicated as an adjunct to diet and physical activity to reduce elevated low-density lipoprotein cholesterol (LDL-C) in patients with primary hyperlipidemia as monotherapy or in combination with HMG-CoA reductase inhibitor.

In the art there are a number of documents describing Sevelamer (hydrochloride or carbonate) tablet formulations. Those tablets are either made by direct compression of a mixture of excipients or by wet granulation using organic solvents or a mixture of organic solvents and water.

WO 98/44933 and WO 00/22008 discloses a tablet made by blending a phosphate binding polymer (sevelamer hydrochloride in the examples) with excipients and compressing said mixture into a tablet. The phosphate binding polymer described should have a particle size smaller than 400 µm and a moisture content of 1-14% in order to obtain a tablet with correct disintegration times, hardness and friability.

WO 01/28527 discloses a process for making a tablet comprising the steps of (1) hydrating sevelamer hydrochloride (2) blending it with other excipients and (3) compressing said mixture to form a tablet core which is coated afterwards. Sevelamer is hydrated to the desired moisture content (5-9%) because according to the invention the compressibility of Sevelamer is strongly dependent of said moisture content.

WO 2006/050315 discloses a tablet of Sevelamer carbonate containing a certain amount of Cl⁻ ions, either in the form of added salts to Sevelamer carbonate, or in the form of a mixture of sevelamer carbonate/sevelamer Hydrochloride. Said tablets are made by a method comprising the steps of: (1) hydrating or drying the aliphatic amine polymer to the desired moisture level; (2) blending the aliphatic amine polymer with any excipients to be included; and (3) compressing the blend using conventional tableting technology.

WO 2010/151439 discloses a tablet made by the process of (a) milling a substantially dry cross-linked polyallylamine salt to a desired particle size, optionally hydrating it, (b) blending with a polyol, and (c) compressing the mixture. It is mentioned that the use of additional water to form the tablet may be very critical as the water added causes significant expansion of the blend volume and causes tablet weight adjustment problems.

In the process for making a solid dosage form by direct compression of a mixture of active ingredient and excipients, the particle size as well as humidity levels of the active ingredient are two key parameters. As seen above this is especially relevant in the case of active ingredients which are affected by humidity, as Sevelamer. Maintaining such parameters into the desired limits is usually difficult for both active ingredient and final dosage form manufacturers leading eventually to quality problems, supply shortage or API storage difficulties.

On the other hand, other documents in the art disclose a wet granulation process using a non-aqueous granulation solvent or a mixture of an organic solvent and water (with a maximum quantity of water in the mixture of 35%) as granulation solvent (WO 2007/094779, EP 1818048 B1, WO 2008/062437, WO 2010/041268, WO 2010/086881). All these documents disclose Sevelamer hydrochloride or carbonate formulations, except WO 2010/041268 which is related to colesevelam hydrochloride. The use of alcohol, or other organic solvents, is needed in order to granulate the mixture prior to compressing it into tablets.

Organic solvents are to be avoided as far as possible in the formulation processes not only for their cost compared with water, but also for their hazardous and in some cases explosive properties and especially for the negative impact on the health of the patients caused by the residual levels present in such dosage forms. Therefore strict quality control measures and monitoring of residual solvents in final dosage form must be applied. Additionally, manufacturing process involves specialised or modified equipment and facilities, such as equipment complying with ATEX directives (equipment and work environment allowed in a work space with an explosive atmosphere).

There are no formulations of Sevelamer using wet granulating solvent systems comprising more than 35% wt of water, let alone using water as the unique granulation solvent. Moreover, the comments in the art regarding wet granulation processes strongly discourages the use of granulating solvent systems comprising high concentrations of water because, according to them, a suitable solid dosage form cannot be obtained. See for instance paragraph 6 of EP 997148 B1 or paragraph 11 of EP 1818048 B1.

Therefore there is a need to provide an alternative pharmaceutical composition making use of a solvent system comprising low amounts of organic solvents or even no organic solvent at all, which is safer for the patient, easy to manufacture, which has excellent product characteristics, such as a wide range of compressibility and rapid disintegration time, and/or allows the use of cross-linked polyallylamine polymer with any moisture content, particle size or physical swelling properties of the cross-linked polyallylamine polymer.

### Detailed description of the invention

Due to the mechanism of action of cross-linked polyallylamine polymers, it is important that once ingested they present as much specific surface as possible to effectively retrieve phosphate anions from the media, therefore the degree and speed of disintegration are basic parameters in oral formulations and those characteristics should be maintained during shelf life and not affected by initial API physic characteristics, formulation process, external storage factors or dosage form used.

It is very important to achieve a solid oral formulation of a cross-linked polyallylamine polymer which is independent of the physical properties of said cross-linked polyallylamine polymer (such as humidity and particle size), readily disintegrable, with good friability and homogeneity, less dependant of external storage conditions and that can be coated with an aqueous based coating without being affected. Also during the whole manufacturing process the formulation should be tolerant to the different humidity of excipients and to changing environmental conditions, such as humidity and temperature, to avoid being affected during each step (granulation, drying, compression, coating, packaging and between steps: storage, transfer from one machine to the other, etc...) in order to have a robust and reproducible formulation which can be easily scaled-up and having same behaviour with different excipients providers, tooling and fabrication sites.

Direct compression is the compactation of a solid mixture, whereas in wet granulation processes a solvent system (either water, organic solvent or a mixture) is used to obtain granules which may be further compressed. Obviously, in wet granulation processes reducing or avoiding the use of organic solvents is a must for the reasons mentioned above.

In some cases wet granulation is preferable compared to direct compression, for instance, in order to make the dosage form more homogeneous, more reproducible and less dependant of the physical properties of the API. Usually granules can be manipulated and stored easier than a solid mixture and have better compaction characteristics as they have better homogeneity and size uniformity than a solid mixture; this fact allows the tableting machine to work at high-speed with minimum deviations in average weight. Usually the friability of the tablets is much better because of the homogenized moisture levels, giving greater cohesion to the tablets which in turn allows using less force at the time of compression to obtain a table with the correct hardness.

The formulation of the present invention overcomes the problems of the prior art by having the aforementioned characteristics.

An object of the present invention is to provide pharmaceutical compositions comprising aqueous wet granulated cross-linked polyallylamine polymers and processes for their preparation.

It has been surprisingly found that during an aqueous wet granulation step, the cross-linked polyallylamine polymers of the present invention suffer a pre-gelatinisation effect, swell and expand but this effect is not noticeable afterwards, once the tablet is obtained. Thus, the produced formulation is more homogeneous, stable, less friable, experiences less expanding, contraction or cracking by the variation in humidity levels or by further aqueous coatings, than the prior art formulations.

The formulation of the present invention is reproducible, cheap, effective, scalable, and can be prepared with standard tooling. Also, it decreases the disintegration time while avoids the use of a monovalent anion source, such as sodium chloride, in sevelamer carbonate tablets. This effect is maintained during storage under standard or accelerated conditions.

The formulation of the present invention is less dependant of the humidity level and particle size of the cross-linked polyallylamine polymer used than the prior art formulations, making the granulation process of the invention highly reproducible and preventing adjusting the quantities of water and excipients.

The formulation of the present invention reduces or avoids the use of organic solvents during manufacturing; it is safe during manufacture, environmentally friendly and safe for the patient

According to an embodiment of the present invention, the cross-linked polyallylamine polymer can be selected from the group consisting of sevelamer, derivatives, mixtures and salts thereof. In a preferred embodiment, the cross-linked polyallylamine polymer is selected from salts of Sevelamer or of a derivative thereof. These salts include addition salts of mineral acids, such as, for example, hydrochloride, hydrobromide, hydroiodide, sulfate, nitrate, phosphate; and addition salts of organic acids such as, for example, acetate, maleate, fumarate, citrate, oxalate, succinate, tartrate, malate, mandelate, methanosulfonate and *p*-toluensulfonate. Preferably, the salt is selected from hydrochloride, carbonate, bicarbonate, acetate or lactate salts, more preferably it is the hydrochloride or carbonate salt. In a preferred embodiment of the invention, the cross-linked polyallylamine polymer is selected from sevelamer hydrochloride, sevelamer carbonate, colesevelam hydrochloride, and mixtures thereof. More preferably the polymer is sevelamer hydrochloride or sevelamer carbonate.

The term sevelamer derivatives refers to polymers that are derivatized at one or more positions. In a particular embodiment, it includes polymers wherein one or more amine groups are alkylated. Preferred alkylating groups include hydrophobic groups (such as aliphatic hydrophobic groups) and/or quaternary ammonium- or amine-substituted alkyl groups. In an embodiment, sevelamer is alkylated with decyl and/or (6-trimehylammonium)hexyl group. In a preferred embodiment of the invention, sevelamer derivative is colesevelam or a salt thereof. More preferably, it is colesevelam hydrochloride.

The wet granulation process of the invention comprises the steps of blending an aqueous granulation solvent system with a cross-linked polyallylamine polymer, and optionally with further pharmaceutically acceptable excipients, granulating the resulting mixture and, optionally, drying the granulate. Said process may additionally comprise a step of blending the granules with further pharmaceutically acceptable excipients.

The wet granulation process according to the present invention provides a formulation comprising the cross-linked polyallylamine polymer, which formulation can be a pre-mix or an intermediate, for example granules, which can then be further processed into a final dosage form such as, for instance, tablets, mini-tablets, Multiple Unit Pellet System (MUPS), films or powders.

The term "granulating solvent system" as used herein refers to a mixture of water and a pharmaceutically acceptable organic solvent miscible in water. In the present invention the granulating solvent system is water or a solvent mixture comprising at least 80% water by weight. Preferably it comprises at least 90% water, at least 95% water, at least 98% water or at least 99% water by weight, or is substantially 100% water.

In an embodiment of the present invention the aqueous granulating solvent system comprises less than 15% organic solvent by weight. Preferably, it comprises less than 15% organic solvent by weight, less than 10% organic solvent, less than 5% organic solvent, less than 2% organic solvent or less than 1% organic solvent by weight. In a preferred embodiment, the aqueous granulating solvent system is free of organic solvents.

In a particular embodiment, the organic solvent, if present, is selected from the group consisting of acetone, methanol, ethanol, isopropyl alcohol, propylene glycol, glycerol and mixtures thereof.

In one embodiment of the invention, the formulation is in the form of tablets, MUPS, mini-tablets, films or powders, which can be obtained by compressing or further processing the granules described above, either alone or in combination with further pharmaceutical excipients.

As used herein, the term "tablet" or "mini-tablet" encompasses compressed pharmaceutical dosage forms of all shapes and sizes, whether coated or uncoated.

In another embodiment of the invention, the formulation is in the form of a sachet, capsule, bag, or bottle, filled with the granules, mini-tablets or powders described above.

In a preferred embodiment of the invention, the formulation is in the form of a tablet prepared by compressing the granules into tablets. The tablet may be further coated using standard techniques known to the skilled person.

In a further preferred embodiment the formulation is a sachet filled with the granules, mini-tablets or powders described above.

In a further embodiment, the pharmaceutical composition according to the present invention can comprise the cross-linked polyallylamine polymer and other drug substances, or active product ingredient (API).

According to one aspect of the present invention, there is provided a process for the preparation of a pharmaceutical formulation comprising a cross-linked polyallylamine polymer comprising the steps of:
a) Providing a cross-linked polyallylamine polymer optionally with one or more pharmaceutically acceptable excipients and/or drug substances,
b) wet granulating the cross-linked polyallylamine polymer of step (a) with water or an aqueous granulating solvent system to form granules, and
c) optionally compressing the resulting granules into tablets, mini-tablets or filling the granules into capsules.

According to another embodiment of the present invention, there is provided a process for the preparation of a pharmaceutical formulation comprising a cross-linked polyallylamine polymer comprising the steps of:
a) Providing a cross-linked polyallylamine polymer optionally with one or more pharmaceutically acceptable excipients and/or drug substances,
b) wet granulating the cross-linked polyallylamine polymer of step (a) with water or an aqueous granulating solvent system to form granules,
a) drying, sieving the granules and optionally blending them with further excipients and optionally sieving again, and
b) optionally compressing the resulting granules into tablets, mini-tablets or filling the granules into capsules.

The mesh sizes used in the sieving are around 100-1000 µm.

Drying can be carried out once or several times, for example in a fluid bed dryer. Drying is performed preferably at temperatures between 20 and 100°C, more preferably between 30 and 60°C, even more preferably between 30 and 40 °C. In a preferred embodiment, the drying step is carried out until moisture content, measured as Loss on Drying (LOD), is between 5 and 25%, more preferably between 7 and 20%, even more preferably between 12 and 16% by weight.

In one embodiment of the invention wet granulation is done by high shear granulation or spray granulation/fluid bed processing method. High shear granulation provides improved cohesiveness of particles, excellent flowability and compression characteristics. High shear granulation is performed using a rapid mixer granulator or planetary mixer. Wet granulation can be done in any equipment suitable for that purpose known to those skilled in the art.

According to a particular embodiment, cross-linked polyallylamine polymer is granulated with a lubricant and, optionally one or more additional pharmaceutical excipients.

Lubricants suitable for pharmaceutical formulations are well-known in the state of the art. In a particular embodiment of the present invention, the lubricant is selected from zinc stearate, stearic acid, magnesium stearate, calcium stearate, polietilenglycol, talc, anhydrous colloidal silica, sodium benzoate and leucine. In a preferred embodiment the lubricant is colloidal silica and/or zinc stearate.

Other pharmaceutical excipients that can be used in the formulation include, among others, disintegrants, fillers, binders, flavours, colorants, anti-tacking agents, sweeteners, glidants, antioxidants, stabilizers, and antiadherents. particularly suitable excipients are starch, sodium starch glycolate, lactose, carboxymethylcellulose sodium, carboxymethylcellulose calcium, polyols, sodium chloride, glucose, sucrose, sorbitol, mannitol, inositol, microcrystalline cellulose, hydroxypropylcellulose (HPC), low-substituted hydroxypropylcellulose (L-HPC), dibasic calcium phosphate, tribasic calcium phosphate, calcium carbonate, magnesium carbonate, dextrines, cellulose, polyvinylpyrrolidone (PVP), polyethylene glycol (PEG), hydroxypropylmethylcellulose (HPMC), ethylcellulose, acacia gum and arabic gum.

In a preferred embodiment of the present invention the cross-linked polyallylamine polymer is sevelamer hydrochloride or sevelamer carbonate.

According to a preferred embodiment of the invention, the process for the preparation of sevelamer pharmaceutical compositions comprises providing a mixture of sevelamer carbonate or sevelamer hydrochloride and a lubricant; granulating said mixture with water or an aqueous granulating solvent system in a high shear mixer. Granulated mass is dried until a Loss on Drying value of from about 7 to 16 % is achieved, preferably from 12 to 16%, and blended with additional pharmaceutical excipients. The final granule may be transferred into a dye and tableted.

The tablets of the present invention may be further coated. Coating can be done in a rotary pan coater, in a fluidised bed apparatus or in any other coating apparatus known in the art.

Typically, the tablet core is contacted with the coating solution until the weight of the tablet core has increased, preferably by amount ranging from about 3 % to 10 % by weight, more preferably from about 4 % to 6 % by weight.

In an embodiment of the invention the coating is an aqueous based composition which, apart from aesthetic reasons, facilitates swallowing and protects the tablet core from external excessive humidity levels and mechanic aggression. Coating can withstand the eventual swelling and contraction of tablet core.

In one embodiment, the coating composition comprises aqueous coatings based in polyvinyl alcohol (PVA), hydroxypropylmethylcellulose (HPMC) or polyvinyl alcohol-polyethylene glycol graft copolymer

In a preferred embodiment coating is the PVA based Opadry® II composition commercialised by Colorcon.

In a preferred embodiment of the present invention, the dosage form is an oval shaped, film coated, compressed tablet with 800 mg of sevelamer carbonate. The pharmaceutical excipients in the tablet are silica colloidal anhydrous, pregelatinized starch, zinc stearate, HPMC and diacetylated monoglyceride.

### Examples

### Example 1: 800 mg sevelamer carbonate aqueous wet granulation tablet core.

816 g sevelamer carbonate, with an average particle size of 57 µm and a moisture content of 2%, measured as Loss on Drying (LOD), and 5 g of colloidal silica were transferred to a high shear mixer and blended. 311 ml of water were added and the mass was granulated.

The granule was dried in a fluid bed to a desired LOD between 12-16% and blended with additional 5 g of colloidal silica, 95 g of pregelatinized starch and 15 g of zinc stearate.

The final granule was then transferred into a dye and tableted at a pressure of about 3 tons in a Manesty eccentric press to a total core weight of 1060 mg.

The resulting white to off-white, oval shaped tablets had 800 mg equivalent to anhydrous sevelamer carbonate, 10 mg of colloidal silica, 95 mg of pregelatinized starch and 15 mg of zinc stearate.

### Examples 2-5: 800 mg sevelamer carbonate and hydrochloride aqueous wet granulation tablets cores.

The procedure of example 1 was followed employing different components or quantities, batch sizes and API characteristics.

The results are shown in the following table, wherein example 1 has also been included.

| **Example n°** | **1** | **2** | **3a** | **3b** | **3c** | **4** | **5** |
|---|---|---|---|---|---|---|---|
| Sevelamer carbonate (mg) | 816 | - | 890 | 840 | 890 | 890 | 890 |
| Sevelamer HCl (mg) | - | 850 | - | - | - | - | - |
| Seveamer d₉₀ (µm) | 134 | 102 | 140 | 140 | 140 | 140 | 140 |
| Sevelamer d₅₀ (µm) | 57 | 43 | 25 | 25 | 25 | 25 | 25 |
| Sevelamer LOD (%) | 2 | 6 | 10 | 5 | 10 | 10 | 10 |
| Water (ml) | 311 | 311 | 311 | 235 | 1400 | 360 | 311 |
| Polyvinylpyrrolidone (mg) | - | - | - | - | - | 7 | - |
| Colloidal Silica (mg) | 10 | 10 | 10 | 10 | 10 | 10 | 10 |
| Pregelatinized starch (mg) | 95 | 95 | 95 | 110 | 95 | 170 | 170 |
| Zinc stearate (mg) | 15 | 15 | 15 | 5 | 15 | 15 | - |
| PEG 4000 (mg) | - | - | - | - | - | - | 20 |

In example 2, Sevelamer hydrochloride has been used. In example 4, an aqueous solution of 2% PVP was the granulation solution. In example 5 PEG was used as lubricant.

In all the preceding examples, the resulting granules were transferred into a dye and tableted at a pressure of about 3 tons in a Manesty eccentric press to obtain a white to off-white, oval shaped tablets with an equivalent to 800 mg anhydrous sevelamer hydrochloride, or carbonate.

### Example 6. Coated tablets with a HPMC based coating

Compressed core tablets prepared as described in example 1 were coated in a coating pan with an aqueous coating solution having a solids content of 10-30 % by weight.

The film coating solution contains a combination of excipients for the aqueous film coating based on pigmented hydroxypropylmethylcellulose (Opadry® white, sold by Colorcon). The coating solution was applied to the compressed cores until a weight gain of approximately 3 to 8 % was achieved.

### Example 7. Coated tablets with a PVA based coating

Compressed core tablets prepared as described in example 1 were coated using a pigmented PVA based coat instead (Opadry® II white, sold by Colorcon) following the procedure of example 6.

The results obtained are disclosed in the following table:

| **Example n°** | **1** | **3a** | **3b** | **3c** | **4** | **5** | **7** |
|---|---|---|---|---|---|---|---|
| Granule LOD (%) | 15 | 10% | 12% | 10% | 10% | 9% | 14% |
| Hardness (Newton) | 150 | 190 | 170 | 195 | 70 | 132 | 160 |
| Disintegration time | < 30s | < 15s | < 10 s | < 15s | < 10 s | < 30 s | 3-4 min |
| Friability (%) | < 0.5 | < 1 | < 1 | < 1 | < 1 | < 1 | N/A |

## Claims

1. Wet granulation process comprising the steps of adding an aqueous granulating solvent system comprising at least 80% water by weight to a cross-linked polyallylamine, or a mixture of excipients comprising said cross-linked polyallylamine; granulating the mixture; and optionally drying the product obtained.

2. Process according to claim 1, which comprises and additional step of blending the granules with further pharmaceutically acceptable excipients.

3. Process according to claim 2, wherein the aqueous granulation solvent system comprises at least 90% water by weight.

4. Process according to claim 3, wherein the aqueous granulation solvent system comprises at least 95% water by weight.

5. Process according to claim 4, wherein the aqueous granulation solvent system comprises at least 98% water by weight.

6. Process according to claim 5, wherein the aqueous granulation solvent system is 100% water.

7. Process according to any of claims 1 to 5, wherein the aqueous granulation solvent system is devoid of organic solvent.

8. Process according to any of claims 1 to 7, wherein the cross-linked polyallylamine polymer is Sevelamer, colesevelam or a salt thereof.

9. Process according to claim 8, wherein the cross-linked polyallylamine polymer is Sevelamer hydrochloride, sevelamer carbonate or a mixture thereof.

10. Formulation comprising a cross-linked polyallylamine obtainable by the wet granulation process of any of claims 1 to 9.

11. Formulation according to claim 10, wherein the cross-linked polyallylamine polymer is Sevelamer hydrochloride, sevelamer carbonate or a mixture thereof.

12. Formulation according to any of claims 10 or 11 which is in the form of a tablet or a mini-tablet.

13. A capsule or a sachet filled with a formulation according to any of claims 10 or 11.
